# EUROPEAN PATENT APPLICATION

(11) **EP 0 845 239 A1**
(43) Date of publication of application: **03.06.1998**
(21) Application number: 97119162.2
(22) Date of filing: 03.11.1997
(51) Int. Cl.: A61B 5/0245, A61B 5/08

(54) **Medical device**

(30) Priority: 25.11.1996 SE 9604320
(71) Applicant: Pacesetter AB, 175 84 Järfälla (SE)
(72) Inventor: Andersson, Jonas, 121 39 Johanneshov (SE); Lidman, Johan, 117 41 Stockholm (SE); Bigert, Carolina, 121 39 Johanneshov (SE)

(57) **Abstract**

A device for determining the respiration rate and/or respiration depth of a patient comprises a sensor (6,8) for sensing heartsounds and an analyzer (20) for analyzing the variation of the amplitude of the sensed heartsounds to determine the respiration rate and/or respiration depth from this amplitude variation. An apparatus for monitoring the respiration of a patient comprises a device of the above mentioned type and the analyzer is arranged to determine a first anomaly in the amplitude variation of the sensed heartsounds as an indication of a respiration anomaly.

## Description

### TECHNICAL FIELD

The present invention relates to a device for determining the respiration rate and/or respiration depth of a patient, comprising a sensor for sensing heartsounds, and an apparatus for monitoring the respiration of a patient, comprising such a device.

### BACKGROUND ART

Sleep apnea is a rather common disorder with diffuse symptoms. Usually during daytime the patient experiences fatigue, concentration problems and problems of staying awake. During night the patient's sleep is disrupted by episodes of apnea, usually caused by the epiglottis falling back and obstructing the airways. The apnea makes the person wake thus disrupting the normal sleep pattern.

Monitoring of the respiration of a patient is needed for several purposes. Sudden infant death syndrome (SIDS) is for example one of the most common causes of death among infants under the age of one year. During night infants normally experience apnea. A healthy infant will wake up to then start breathing if the apnea is too long. If the infant is unable to wake itself up, however, accidental suffocation and sudden death can occur. The reason for the inability of some infants to wake themselves up and the etiology of SIDS is to a large extent unknown but some correlation to rotavirus infection has been found. The clinical manifestation consists , as mentioned, in stops in the breathing of the infant during sleep and as a consequence death of the infant.

The usual way of investigating apnea is to monitor respiration and blood oxygen saturation during sleep. The respiration signal will indicate the episodes of apnea, while the blood oxygen signal indicates the severity of the apnea.

Several respiration monitoring systems are known. Thus in e.g. US,A,5 143 078 a monitoring system is described to determine a patient*'*s respiration rate from breath sounds by an appropriate sensor, like a microphone. Special means are provided for reducing the effects of heart sounds and ambient noise.

An infant health monitoring system is disclosed in US,A,5 479 932 in which, in addition to respiration of the infant, also large motor movement and heart beats are detected simultaneously and an alarm signal is generated only when anomalies are found in all three abovementioned health related conditions. In this way false alarms are eliminated.

A sudden infant death syndrome monitor and stimulator is described in US,A,5 515 865. Movements and acoustic activity, e.g. heartbeats and breathing, are then detected and a stimulator to stimulate movement activity or acoustic activity in the infant is operated when no movement activity and no acoustic activity are detected.

In US,A,5 105 354 a method and apparatus is disclosed for forecasting sudden infant death syndrome by utilizing a characteristic change in the correlation between respiration and heart beats of infants which occurs a few minutes before the start of a sleep-apnea.

EP, A2, 0 504 945 discloses an apparatus for diagnosing and quantitatively analysing apnea by recording the heart rate by ECG electrodes, respiration and snoring sounds by a microphone, the blood oxygen saturation degree by an oxymeter and the position of the patient by a suitable position sensor. The recorded data are transferred to a computer for further analysis.

A new way of determining the respiration rate and to some degree the respiration depth of a patient has now been proposed which is based on the fact that the amplitude of heartsounds varies in a characteristic manner during the respiration cycle. One reason for this phenomenon is that the respiration changes the geometry of the chest, which means that the mechanical coupling between the heart and the sensor is changed, which in its turn changes the amplitude of the heartsounds picked up by the sensor. Another reason is that the filling of the heart with blood is depending on the pressure in the thorax and the respiration influences this pressure. The filling of the heart in its turn influences the strength of the heart beats and this strength affects the amplitude of the heartsounds.

### DISCLOSURE OF THE INVENTION

The purpose of the present invention is consequently to provide a simple and reliable device for determining the respiration rate and to some degree the respiration depth of a patient by utilizing the amplitude variation of heartsounds.

This purpose is obtained, according to the invention, by a device comprising a sensor for sensing heartsounds, and characterized by an analyzer for analyzing the variation of the amplitud of the sensed heartsounds to determine the respiration rate and respiration depth from this amplitude variation.

Another purpose of the invention is to provide an apparatus for monitoring the respiration of a patient comprising such a device.

This purpose is obtained, according to the invention, by a respiration monitoring apparatus which is characterized in that the analyzer is arranged to determine a first anomaly in the amplitude variation as an indication of a respiration anomaly.

According to other advantageous embodiments of the apparatus according to the invention a second sensor is provided for measuring the degree of blood oxygen saturation to be supplied to the analyzer for analysis together with sensed heart sound data and a body movement sensor can be provided to sense body movements of the patient for supplying sensed body movement data to the analyzer for analysis together with other sensed data. By adding these additional data to the sensed heart sound data a more reliable and thorough investigation or diagnosis of the respiration can be made.

According to another advantageous embodiment of the apparatus the analyzer is arranged to trigger a first alarm in response to the determination of a respiration anomaly giving nursing staff or parents of an infant a possibility to intervene. Just waking up the patient may often be enough for the patient to regain breathing.

According to yet other advantageous embodiments of the apparatus according to the invention the analyzer is arranged to determine a second anomaly in the amplitude variation of the sensed heart sounds as an indication of an anomaly other than the respiration anomaly. This other anomaly can be e.g. no heartsounds being detected, which then probably is due to a dislocation of the sensor. It is thus possible to monitor also the correct functioning of the apparatus. The analyser is then preferably arranged to trigger a second alarm in response to the determination of said other anomaly. The first and second alarms can suitably be realized by an alarm means controlled by the analyzer to deliver acoustic and/or visual signals and/or mechanical vibrationsignals of two different qualities.

According to still other advantageous embodiments of the apparatus according to the invention the sensor comprises an accelerometer, disposed to detect heartsounds. The accelerometer can preferably comprise a piezoelectric element with amplifier and signal pre-conditioning means. These signal pre-conditioning means can comprise high pass filtering, rectifying and signal smoothing means. By choosing the cut-off frequency of the high pass filtering means to about 100 Hz, frequencies originating from body movements of the patient, normally in the range of 1 - 5 Hz, are removed, thus making the functioning of the apparatus reliable also when the patient is moving.

According to another advantageous embodiment of the apparatus according to the invention the sensor and the analyzer are mounted in a pacemaker. In this way a rate response pacemaker, based on the respiration, is obtained.

According to yet another advantageous embodiment of the apparatus according to the invention the heartsound sensor, the analyzer and electric supply means are located in a common housing having a replaceable sticky surface layer for attachment to the skin of the patient*'*s chest. The housing can have the shape of a disc with a diameter of about 30 mm and a thickness of a few mm. It allows for change of battery and can be carried by the patient without any discomfort.

According to still other advantageous embodiments of the apparatus according to the invention said heartsound sensor is contained in a recording device also comprising memory means for storing sensed heartsound data for subsequent transfer to the analyzer which is separated from said device, said recording device having a sticky surface layer for attachement to the skin of the patient's chest. A blood oxygen saturation sensor and/or a body movement sensor can be connected to said memory means for transmission of measured data via a radio frequency, light or wire communication link. The analyzer comprises a data processing unit, like a computor, and is designed as a stationary equipment intended to be placed in a hospital. In this way all recordings of data could be performed by the patient at home, contrary to the investigation possibilities of e.g. sleep apnea offered by previously known techniques which require a one-night stay at a hospital, which is a costly procedure. Furthermore the previously known equipments are bulky and often interfere with the patient under investigation thus giving an unreliable diagnosis. The above mentioned embodiments of the apparatus according to the invention are small apparatus which will interfere minimally with the normal sleep of the patient can be manufactured at a relatively low cost and for the analysis existing personal computors can be used. The recording device carried by the patient can even be designed for being directly plugged into an ordinary PCMCIA input adapter of personal computers for transfer of data stored in the memory means of the recording device to the computer for analysis.

### BRIEF DESCRIPTION OF THE DRAWINGS

The invention will be described more in detail with reference to the drawings on which
Figures 1 and 2 show diagrams illustrating the relation between sensed heart signals and the respiration,
Figure 3 shows a pacemaker having a sensor with associated electronics used in the device and apparatus according to the invention,
Figure 4 shows the sensor in figure 3 more in detail,
Figure 5 shows a block diagram of an embodiment of the apparatus according to the invention, and
Figure 6 shows an embodiment in which the analyzer is separated from the recording and storing device.

### DESCRIPTION OF A PREFERRED EMBODIMENT

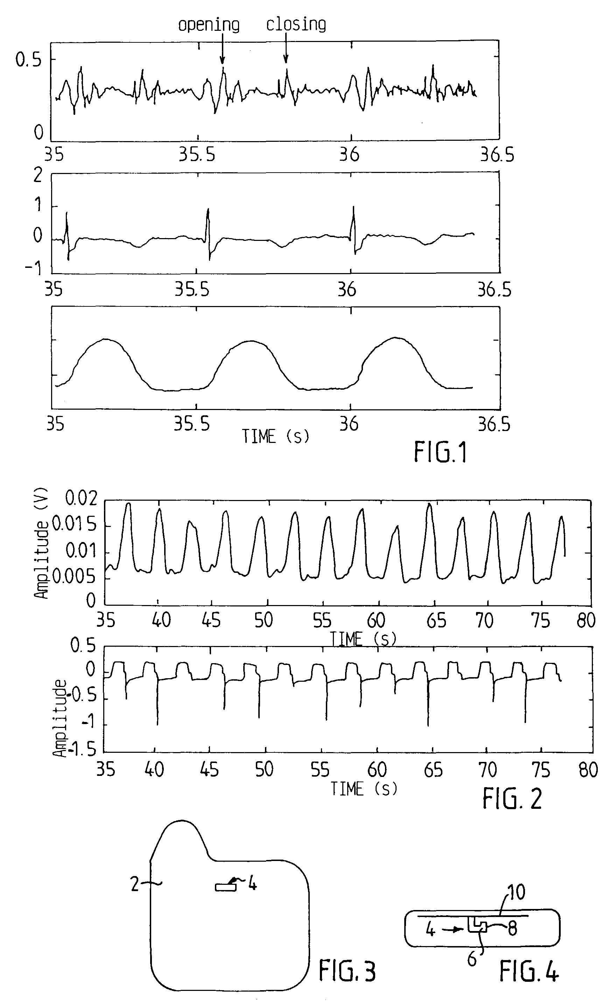

Figure 1 shows the heart sound activity, picked up by an accelerometer and amplified, a surface ECG, and the left ventricular pressure respectively, as functions of time. The aortic valves opening and closing can be clearly observed in the accelerometer signal and are indicated in the corresponding curve. From figure 1 it appears that the amplitude of the heartsounds varies with the respiration. This appears still more clear from figure 2.

Figure 2 shows the accelerometer signal after high pass filtering, the cut-off frequency being 100 Hz, rectifying and smoothing, and the signal out of a respirator determining the respiration in this case, respectively. As appears from these diagrams the respiration frequency is very well reproduced in the sensed heartsound signal. Thus there is a very close correlation between the mechanical heart activity and the respiration and the respiration rate can be directly extracted from the heartsound curve.

As mentioned above the invention can be used in a pacemaker to provide a rate response pacemaker, based on the respiration of the patient and figure 3 shows a pacemaker 2 with an accelerometer 4 mounted inside the pacemaker can.

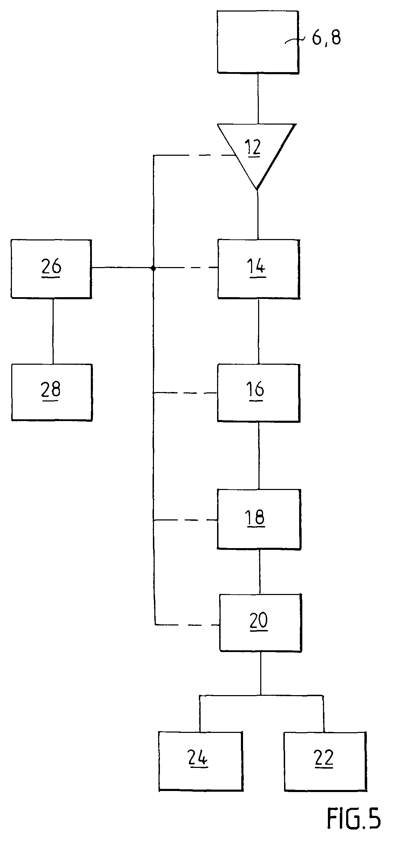

The accelerometer 4, which is shown more in detail in figure 4, is of a piezoelectric type. It comprises a L-shaped piezoelectric rod 6 having a weight 8 fixed at a free end of the rod 6. The other end of the rod 6 is attached to a printed circuit board 10 comprising the associated electronics. The electronics comprise a non-inverting amplifier 12 with a lower cut-off frequency of about 20 Hz and a gain of 2000, a high pass filter 14, rectifying means 16, signal smoothing means 18 and an analyzer 20, see figure 5. Thus the signal from the piezoelectric element 6 is first amplified and then high pass filtered. The cut-off frequency of the high pass filter 14 is chosen to 100 Hz. Frequencies related to body movements of the patient are then removed from the sensed signal which makes the device and the apparatus function reliably also when carried by a patient in motion. The analyzer 20 comprises a data processor, like a computer.

If normal variation is detected in the amplitude of the sensed heartsound signal the apparatus gives an "OK" audio signal. If heartsounds with too low amplitude variation is detected to last beyond a periode of time of predetermined length a first alarm 22, preferably a high intensity audio buzzer, is triggered to indicate an apnea. If no heartsounds are sensed during a predetermined time, a second alarm 24 is triggered to indicate a failure, probably due to dislocation of the sensor. These two alarms can preferably be realized by one and the same alarm means disposed to deliver acoustic and/or visual signals and/or mechanical vibrationsignals of two different qualities.

The necessery electric supply to the electronics is delivered by a battery 26 provided with a low battery guard 28.

The device according to the invention can be mounted inside a pacemaker can as described above. It can, however, also be mounted in a housing, intend to be attached to the skin of the patient*'*s chest. The device is then disc-shaped with a diameter of 30 mm and a thickness of a few mm. To allow for change of battery the housing has a replaceable, preferably sticky surface layer for attachment to the skin.

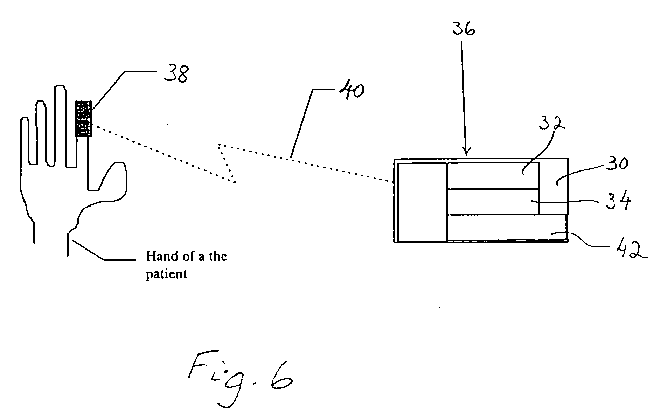

Figure 6 shows an embodiment with a recording and storing device 30 comprising a heartsound sensor in the form of a piezoelectric sensor 32, and memory means 34 for storing sensed data. The device 30 is inteded to be attached to the skin of the chest wall of the patient by a sticky pad, at the side 36, with the piezoelectric sensor 32 adjacent to the chest wall. A second sensor in the form of a finger oxygen sensor 38 is intended to be attached to the finger of a patient to continuously measure the degree of blood oxygen saturation. This sensor 38 is suitably a light emitting oxygen measuring sensor and this sensor is connected to the memory means 34 of the device 30 via a radio frequency telemetry link or a light or wire communication link, indicated at 40.

In addition, data from a body movement sensor (not shown in the figure), can also be supplied to the memory means 34.

After the data collection or recording phase, which is suitably performed by the patient at home, the device 30 is sent, e.g. by mail, to a hospital, where an analyzer is available. The data stored in the memory means 34 is then transferred to the computer of the analyzer. For that purpose the device 30 can be designed for being directly plugged into an ordinaly PCMCIA adapter of the computer.

The device 30 can preferably comprise a data compression stage 42 for facilitating storage, transfer and even analysis of large volumes of data.

By simultanous recording of respiration information by heartsound sensing, degree of blood oxygen saturation and body movements for analysis more accurate assessment and reliable diagnosis of respiration disorders are secured.

## Claims

1. A device for determining the respiration rate and/or respiration depth of a patient, comprising a sensor (6,8,32) for sensing heartsounds, **characterized by** an analyzer (20) for analyzing the variability of the amplitude of the sensed heartsounds to determine the respiration rate and/or respiration depth from this amplitude variation.

2. The device according to claim 1, **characterized in** that the analyzer (20) is arranged to determine the passage of a predetermined threshold value of a predetermined flank of the amplitude variation curve, obtained from the sensed heartsounds.

3. An apparatus for monitoring the respiration of a patient, comprising a device (6,8,32) for determining the respiration rate and/or respiration depth of a patient according to claims 1 or 2, **characterized in** that the analyzer (20) is arranged to determine a first anomaly in the amplitude variation of the sensed heartsounds as an indication of a respiration anomaly.

4. The apparatus according to claim 3, **characterized in** that the analyzer (20) is arranged to determine the decrease below prescribed limits of the amplitude variation during a predetermined periode of time as a respiration anomaly.

5. The apparatus according to claim 4, **characterized in** that the analyzer (20) is arranged to continously compare the amplitude of the sensed heartsounds with predetermined threshold values to detect a decrease of the amplitude variation below the prescribed limits.

6. The apparatus according to any of the claims 3 through 5, **characterized in** that the analyzer (20) is arranged to appoint too low a respiration rate as a respiration anomaly.

7. The apparatus according to any of the claims 3 through 6, **characterized in** that a second sensor (38) is provided for measuring the degree of blood oxygen saturation to be supplied to the analyzer for analysis together with sensed heartsound data.

8. The apparatus according to any of the claims 3 through 7, **characterized in** that a body movement sensor is provided to sense body movements of the patient for supplying sensed body movement data to the analyzer for analysis together with other sensed data.

9. The apparatus according to any of the claims 3 through 8, **characterized in** that the analyzer (20) is arranged to trigger a first alarm (22) in response to the determination of a respiration anomaly.

10. The apparatus according to any of the claims 3 through 7, **characterized in** that the analyzer (20) is arranged to determine a second anomaly other than the respiration anomaly from the sensed data.

11. The apparatus according to claim 10, **characterized in** that the analyzer (20) is arranged to trigger a second alarm (24) in response to the determination of said other anomaly.

12. The apparatus according to any of the claims 9 through 11, **characterized in** that an alarm means is controlled by the analyzer (20) to deliver acoustic and/or visual signals and/or mechanical vibration signals of two different qualities to form said first and second alarms.

13. The apparatus according to any of the claims 3 through 10, **characterized in** that the heartsound sensor comprises an accelerometer (4).

14. The apparatus according to claim 13, **characterized in** that the accelerometer (4) comprises a piezoelectric element (6) with amplifier (12) and signal pre-conditioning means (14,16,18).

15. The apparatus according to any of the claims 1 through 11, **characteried in** the sensor and the analyzer are mounted in a pacemaker (2).

16. The apparatus according to any of the claims 3 through 14, **characterized in** that the heartsound sensor, the analyzer and electric supply means are located in a common housing having a replaceable sticky surface layer for attachment to the skin of the patient*'*s chest.

17. The apparatus according to claim 16, **characterized in** that said electric supply means comprise a battery (26) and a low battery guard (28).

18. The apparatus according any of the claims 3 through 8, **characterized in** that said heartsound sensor (32) is contained in a recording device (30) also comprising memory means (34) for storing sensed heartsound data for subsequent transfer to the analyzer which is separated from said device, said recording device having a sticky surface layer (at 36) for attachement to the skin of the patient's chest.

19. The apparatus according to claims 7 and 18, **characterized in** that said blood oxygen saturation sensor (38) is connected to said memory means (34) for storing measured blood oxygen saturation data.

20. The apparatus according to claim 19, **characterized in** that said blood oxygen saturation sensor (38) is connected to said memory means (34) via a radio frequency (40), light or wire communication link.

21. The apparatus accoridng to claim 14 and any of the claims 18 through 20, **characterized in** that said piezoelectric element is provided to also measure body movements of the patient for supplying also these data to the memory means (34) for storage and subsequent analysis.

22. The apparatus according to any of the claims 18 through 21, **characterized in** that said recording device (30) comprises data compression means (42) for compressing measured data.

23. The apparatus according to any of the claims 18 through 22 **characterized in** that the analyzer comprises a data processing unit, like a computer, and is designed as a stationary equipment.
